# EUROPEAN PATENT APPLICATION

(11) **EP 3 528 179 A1**
(43) Date of publication of application: **21.08.2019**
(21) Application number: 18156991.4
(22) Date of filing: 15.02.2018
(51) Int. Cl.: G06N 3/04, G06N 3/08, A61B 5/00, G16H 50/00

(54) **TRAINING A NEURAL NETWORK**

(71) Applicant: Koninklijke Philips N.V., 5656 AE Eindhoven (NL)
(72) Inventor: MANKE, Dirk, 5656 AE Eindhoven (NL)
(74) Representative: de Haan, Poul Erik

(57) **Abstract**

A method (100) performed by a central processor of training a first neural network to classify medical data comprises receiving (102) a first parameter indicative of a weight in the first neural network, the first parameter being obtained during training of the first neural network on a first set of medical data. The method further comprises combining (104) the first parameter with a second parameter to determine an update to the weight, the second parameter being indicative of a respective weight in a second neural network. The method (100) further comprises sending (106) the update to the weight to the first neural network.

## Description

### FIELD OF THE INVENTION

The disclosure relates to machine learning and more specifically, but non-exclusively, to training a neural network for classifying medical data.

### BACKGROUND OF THE INVENTION

Recent advances in machine learning and deep learning have demonstrated the potential of the application of neural networks (or deep neural networks) for medical image analysis tasks. Examples of tasks that are well suited to machine learning applications include anatomy detection in images, anomaly detection in images, and automatic adjustment of imaging system geometries to individual patients based on e.g. optical range camera data that is used to sense a patient's size and shape.

A basic challenge of deep learning is the huge amount of annotated training data required for training of a neural network. In addition, data privacy principles and regulations may impede the transfer of patient data from a clinical site to an external central server of the manufacturer of the medical device.

### SUMMARY OF THE INVENTION

As noted above, one of the challenges associated with the use of deep learning is that in order to produce a neural network model capable of classifying data with a high degree of accuracy, for most tasks, the neural network model typically needs to be trained on large quantities of annotated training data examples. In the field of medical imaging, at least one challenge this poses is the collection and/or distribution of medical data (e.g. transmission of medical data between sites), which is often restricted due to data privacy regulations. This may have a large impact on the accuracy of neural network models if insufficient data examples can be made available to the neural network during training.

According to a first aspect there is provided a method performed by a central processor of training a first neural network to classify medical data. The method comprises receiving a first parameter indicative of a weight in the first neural network, the first parameter being obtained during training of the first neural network on a first set of medical data. The method further comprises combining the first parameter with a second parameter to determine an update to the weight, the second parameter being indicative of a respective weight in a second neural network, and sending the update to the weight to the first neural network.

In this way a processor (such as a central processor) may collect values of weights (or other indications of the values of the weights, such as changes in weight values) from first and second local instances of a neural network each time one or more of the local instances is trained on a set of medical data. The values from the first and second neural networks may be combined and the combined values sent back to the first neural network to enable the first neural network to update its weights based on the combined learning experiences of both the first and second neural networks. Essentially therefore, the central processor acts to collate weight values and thus learning insights from a plurality of local instances of a neural network, without the medical data used to train the local neural networks needing to be transferred or combined into a single database. In this way, private or confidential medical data can be used to train neural networks that are located remotely (e.g. on different apparatuses or data storage networks), thus increasing the amount of training data that can be made available and increasing the accuracy of the resulting model without having to transfer the confidential medical data between clinical sites.

According to a second aspect there is provided a method of training a first neural network to classify medical data. The method comprises training the first neural network on a first set of medical data, and sending a first parameter indicative of a weight in the first neural network to a central processor, the first parameter being obtained during the training of the first neural network. The method further comprises receiving an update to the weight from the central processor, the update comprising a combination of the first parameter with a second parameter, the second parameter being indicative of a respective weight in a second neural network, and updating the weight in the first neural network, based on the received update.

According to a third aspect there is provided a system configured for training a first neural network to classify medical data. The system comprises a memory comprising instruction data representing a set of instructions. The system further comprises a processor configured to communicate with the memory and to execute the set of instructions. The set of instructions, when executed by the processor, cause the processor to receive a first parameter indicative of a weight in the first neural network, the first parameter being obtained during training of the first neural network on a first set of medical data, combine the first parameter with a second parameter to determine an update to the weight, the second parameter being indicative of a respective weight in a second neural network, and send the update to the weight to the first neural network.

According to a fourth aspect there is provided a system configured for training a first neural network to classify medical data. The system comprises a memory comprising instruction data representing a set of instructions, a processor configured to communicate with the memory and to execute the set of instructions. The set of instructions, when executed by the processor, cause the processor to train the first neural network on a first set of medical data, send a first parameter indicative of a weight in the first neural network to a central processor, the first parameter being obtained during the training of the first neural network, receive an update to the weight from the central processor, the update comprising a combination of the first parameter with a second parameter, the second parameter being indicative of a respective weight in a second neural network, and update the weight in the first neural network, based on the received update.

According to a fifth aspect there is provided a computer program product comprising a non-transitory computer readable medium, the computer readable medium having computer readable code embodied therein, the computer readable code being configured such that, on execution by a suitable computer or processor, the computer or processor is caused to perform any one of the methods herein.

These and other aspects of the invention will be apparent from and elucidated with reference to the embodiments described hereinafter.

### BRIEF DESCRIPTION OF THE DRAWINGS

For a better understanding of the disclosure, and to show more clearly how it may be carried into effect, reference will now be made, by way of example only, to the accompanying drawings, in which:
Figure 1 shows an example method performed by a central processor according to some embodiments;
Figure 2 shows an example method of training a first neural network to classify medical data according to some embodiments;
Figure 3 shows an example system for training neural networks to classify medical data according to some embodiments;
Figure 4 shows an example system configured for training a first neural network to classify medical data according to some embodiments; and
Figure 5 shows an example system configured for training a first neural network to classify medical data according to some embodiments.

### DETAILED DESCRIPTION OF EMBODIMENTS

Large amounts of training data is typically needed to train a neural network model. In the case of confidential medical data, data privacy concerns and/or regulations often mean that medical data cannot be transferred between sites and is thus only accessible to a particular data storage network (e.g. computer network).

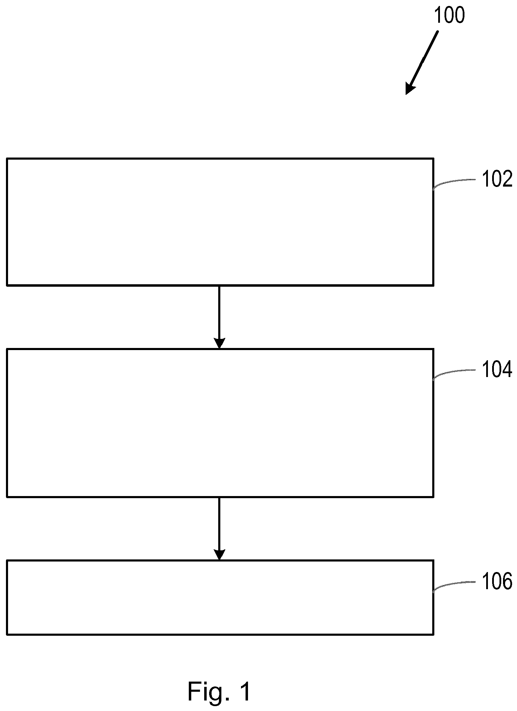

Figure 1 shows a method 100 performed by a central processor, of training a first neural network to classify (e.g. process, annotate, recognize or localize a feature of, or otherwise classify) medical data according to some embodiments herein. According to a first step 102, the method comprises receiving a first parameter indicative of a weight in the first neural network, the first parameter being obtained during training of the first neural network on a first set of medical data. In a second step 104, the method comprises combining the first parameter with a second parameter to determine an update to the weight, the second parameter being indicative of a respective weight in a second neural network. In a third step 106 the method comprises sending the update to the weight to the first neural network.

The first neural network is thus trained on the first set of medical data and sends a parameter comprising the results of the training (e.g. a parameter indicative of a weight value) to the central processor. The central processor combines this parameter with an equivalent parameter from a second neural network and sends an update to the first neural network. In this way the values of weights of the second neural network are shared and combined with the first neural network. The first neural network may thus take the benefit of the learning experiences of the second neural network, without having to be trained on the same data as the second neural network.

Artificial neural networks or, simply, neural networks, will be familiar to those skilled in the art, but in brief, a neural network is a type of model that can be used to classify medical data (for example, classify, or identify the contents of medical data such as medical image data). The structure of a neural network is inspired by the human brain. Neural networks are comprised of layers, each layer comprising a plurality of neurons. Each neuron comprises a mathematical operation. In the process of classifying a piece of data, the mathematical operation of each neuron is performed on the data to produce a numerical output, and the outputs of each layer in the neural network are fed into the next layer sequentially. Generally, the mathematical operations associated with each neuron comprise one or more weights that are tuned during the training process (e.g. the values of the weights are updated during the training process to tune the model to produce more accurate classifications).

For example, in a neural network model for classifying the contents of images, each neuron in the neural network may comprise a mathematical operation comprising a weighted linear sum of the pixel (or in three dimensions, voxel) values in the image followed by a non-linear transformation. Examples of non-linear transformations used in neural networks include sigmoid functions, the hyperbolic tangent function and the rectified linear function. The neurons in each layer of the neural network generally comprise a different weighted combination of a single type of transformation (e.g. the same type of transformation, sigmoid etc. but with different weightings). As will be familiar to the skilled person, in some layers, the same weights may be applied by each neuron in the linear sum; this applies, for example, in the case of a convolution layer. The weights associated with each neuron may make certain features more prominent (or conversely less prominent) in the classification process than other features and thus adjusting the weights of neurons in the training process trains the neural network to place increased significance on specific features when classifying an image. Generally, neural networks may have weights associated with neurons and/or weights between neurons (e.g. that modify data values passing between neurons).

During training, the neural network processes examples of data that have been processed (e.g. annotated or classified) by a human annotator (training data). For each piece of training data, the neural network processes the training data, compares the classification produced by the neural network with the classification provided by the human annotator and updates the weights in order to improve the classification.

Generally, the first neural network and the second neural network may comprise any type of neural network that comprises weights and that is suitable for processing medical data. Examples of suitable neural networks include, but are not limited to feed forward models (such as convolutional neural networks, autoencoder neural network models, probabilistic neural network models and time delay neural network models), radial basis function network models, recurrent neural network models (such as fully recurrent models, Hopfield models, or Boltzmann machine models), or any other type of neural network model comprising weights.

Generally, the first and second models comprise the same type of model. For example, the first and second models may be local instances (e.g. local copies) of a common neural network model. As such, the first and second neural network models may comprise the same number of layers, each layer comprising the same type of mathematical functions. Furthermore, each weight in the first neural network may have a respective or equivalent weight in the second neural network model, that weighs a respective or equivalent mathematical function in a respective or equivalent part of the structure of the second neural network model.

The first and second neural network models may be used to classify medical data. The medical data may be any type of medical data, such as medical data comprising images (e.g. medical imaging data), data comprising text such as documents or records, audio data or any other type of medical data that can be classified by first and second neural network models. Examples of medical data include medical images (e.g. x-ray images, ultrasound images, CT images, MR images or optical images of the exterior of the patient etc.), medical records, test results, acquisition parameters, manually entered user data, anthropometric patient data (e.g. patient size, weight), or data from additional sensors (e.g. optical 3D range cameras), and/or any other types of medical data or medical notes.

As noted above, the method 100 is a method performed by a central processor. In this sense, the central processor may be considered to be central in the sense that the central processor may be in communication with both the first and second neural network models. The skilled person will appreciate that central in this context does not necessarily mean geographically central. In some embodiments, the central processor may comprise, for example, a server such as a remote server, a desk top computer, or a processor (or one or more distributed processors) associated with cloud computing. In some embodiments, the central processor may be thought of as a central parameter server in the sense that in some embodiments, the central processor (as will be explained in detail below) may co-ordinate or collate parameters associated with the weights of the first, second (and any subsequent) neural networks.

In some embodiments, the central processor may be associated with a memory and/or data storage for storing and/or accessing a copy (e.g. another instance) of the same type of neural network model as the first and second neural network models (e.g. the central processor may access its own local copy of the "common" neural network model referred to above). Such a copy of the neural network model, accessible to the central processor, is referred to herein as a central neural network model. The central processor may update the parameters of the central neural network model with any updates to the weights. In this way, the central neural network model may comprise a central copy of the current "best" weights from the combining the weights of the first, second (and any subsequent) neural network models.

In some embodiments, the first neural network may be located (e.g. stored) remotely to the central processor. For example, the first neural network may be located in a different computing network (e.g. such as a different data storage network, local area network (LAN), server or device) to the central processor. In some embodiments, the second neural network may be located in a different location to the first neural network and/or the central processor. For example, the second neural network may be located in a second computing network (e.g. such as a second data storage network, local area network (LAN), server or device).

Generally, the first neural network is trained on a first set of medical data and the second neural network is trained on a second set of medical data. The first set of medical data may be stored on a separate network (e.g. computing network or data storage network) or apparatus to the central processor and/or the second neural network, such that the first set of medical data is inaccessible to the central processor and/or the second neural network (e.g. inaccessible without the first set of medical data being transmitted or sent to the central processor and/or the second neural network). In some embodiments the second set of medical data is stored on a separate data storage network or apparatus to the central processor and the first neural network, such that the second set of medical data is inaccessible to the central processor and the first neural network (e.g. inaccessible without the second set of medical data being transmitted or sent to the central processor and/or the first neural network).

In some embodiments the first set of medical data comprises confidential data that cannot be transmitted to the central processor or the second neural network due to data privacy considerations (e.g. data privacy laws). In effect, such data privacy considerations or restrictions on data sharing may prevent the first set of medical data from being combined with the second set of medical data to form a combined set of medical data that can be used to train a neural network. The first and second sets of medical data may thus be confined to specific locations.

Turning back to Figure 1, the step of receiving 102 a first parameter indicative of a weight in the first neural network, may comprise receiving the first parameter from the first neural network. The first parameter may be produced by the first neural network during (e.g. as a result of) training of the first neural network on the first set of medical data. The parameter may comprise, for example, any one or combination of ones of: the value of the weight after the training, a change in value (or gradient) of the weight reflecting the change in value that occurred during the training (e.g. the value before training subtracted from the value after training), or the change in value of the weight compared to a value of the weight stored remotely that is accessible to the central processor (e.g. stored in a central neural network model as described above). Sending changes to weights (e.g. gradients) rather than the weight values themselves may reduce the computational overhead associated with transmissions to the central processor.

It will be appreciated that the step of receiving 102 may comprise receiving a plurality of parameters indicative of a plurality of weights in the first neural network. In some embodiments, the step of receiving 102 may comprise receiving a parameter set comprising values indicative of all of the weights of the first neural network.

In some embodiments, the method may further comprise receiving the second parameter from the second neural network. The second parameter may comprise any of the parameter types listed above with respect to the first parameter.

The step of combining 104 the first parameter with a second parameter may comprise taking an average or a weighted average of the first and second parameters. The value of the weighting in a weighted average calculation may enable the values of the combined weights to converge more quickly, e.g. by preventing large changes or "swings" in the values of the weights caused by outlying or anomalous data.

The step of sending 106 the update to the weight to the first neural network may comprise transmitting an updated parameter to the first neural network. This may be used by the first neural network to update the value of the weight in the first neural network, effectively updating the value of the weight with the insights gained by the second neural network. In this way, the learning experiences of the second neural network gained on the second set of medical data may be combined with the learning experiences of the first neural network gained on the first set of medical data to speed up the learning process of the first neural network.

In some embodiments, the method may further comprise sending the update to the weight to the second neural network. In this way, the second neural network may also benefit from the learnt parameter/weight values of the first neural network.

It will be apparent that the method above may be extended to any number of local instances of a neural network. For example, the step of receiving 102 may further comprise receiving further parameters indicative of the weight in third or subsequent neural networks, the further parameters being obtained during training of the third or subsequent neural networks on third or subsequent sets of medical data. The step of combining 104 may then comprise combining the first parameter, the second parameter and the third or subsequent parameters to determine an update to the weight. The step of sending 106 may comprise sending the update to the weight to the first, second and third or subsequent neural networks.

In some embodiments the method further comprises repeating the steps of receiving 102, combining 104 and sending 106 until a quality criterion associated with the first neural network is satisfied. For example, these steps may be repeated until the first neural network reaches a threshold accuracy. The accuracy may be determined, for example, by determining the percentage of correct classifications made by the first neural network on a quality control set of example medical data (e.g. a set of unseen medical data used specifically for quality control purposes). The steps may also be repeated until the value of the weight converges within a threshold (e.g. until further training changes the values of the weights by less than a predefined threshold change indicating that the weight values are converging towards optimal values.)

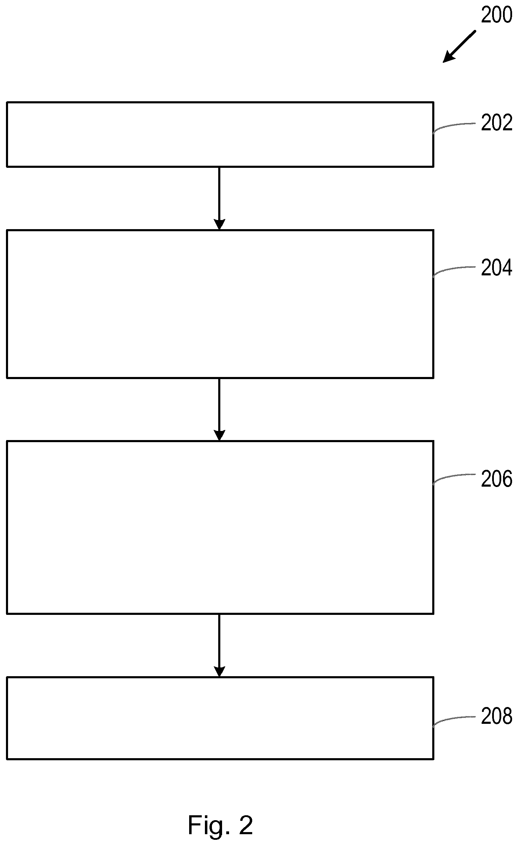

Turning now to Figure 2, there is a method 200 of training a first neural network to classify medical data. The method 200 comprises in a first step 202 training the first neural network on a first set of medical data. In a second step 204, the method 200 comprises sending a first parameter indicative of a weight in the first neural network to a central processor, the first parameter being obtained during the training of the first neural network. In a third step 206 the method 200 comprises receiving an update to the weight from the central processor, the update comprising a combination of the first parameter with a second parameter, the second parameter being indicative of a respective weight in a second neural network. In a fourth step 208 the method comprises updating the weight in the first neural network, based on the received update.

In this way, the first neural network is trained on the first set of medical data, and information indicative of the value of a weight as a result of this training is sent to a central processor which combines the update with an update to the same weight from a second neural network. This update is then received by the first neural network so that the weight in the first neural network can be updated based on the combined learning insights of the first neural network and the second neural network, without the first neural network having access to the training data of the second neural network.

The first and second neural networks, the first set of medical data, the second set of medical data, the central processor and the first and second parameters were described above with respect to method 100 and the details therein will be understood to apply here.

For example, as described above, in some embodiments, the first set of medical data is stored on a separate network (e.g. separate data storage network or computer network) or apparatus to the central processor, such that the first set of medical data is inaccessible (e.g. without the first set of data being copied or transmitted) to the central processor.

In some embodiments, the second neural network is trained on a second set of medical data that is stored on a separate network (e.g. separate data storage network or computer network) or apparatus to the central processor and the first neural network, such that the second set of medical data is inaccessible to the central processor and the first neural network.

The first set of medical data and/or the second set of medical data may comprise confidential data (e.g. such as data identifying the patient) that cannot be transmitted to the central processor or the second neural network due to data privacy considerations.

In some embodiments, the step 202 of training the first neural network on a first set of medical data comprises training the first neural network to classify or locate an object in a medical image. For example the first neural network may be used to determine the contents of the medical image, to make a diagnosis of a medical condition based on the medical image or to detect an abnormality (e.g. a lung tumor or a pneumothorax in chest images).

In some embodiments, the step of training comprises training the first neural network to determine one or more settings for medical equipment, based on one or more patient attributes. For example, the first neural network may be trained to determine an appropriate geometric setup for medical imaging equipment. For example, based on 3D range camera data that images the patient, the first neural network may be trained to determine an appropriate detector height adjustment and/or collimation for an x-ray imager in order correctly image a patient's chest whilst they are standing upright. In such an embodiment, the patient's size and shape may be determined by the neural network and this may be used to produce geometry and collimation settings appropriate for the individual patient. In this embodiment, the training data may comprise 3D range camera data (as example inputs to the model), the x-ray imaging parameters (e.g. geometry and/or collimation settings that the model is to predict) and the corresponding X-ray image. In this context, the X-ray image provides information about the real position of the lung field, e.g. knowledge about the internal anatomy of the patient, and therefore serves as the ground truth for the learning process.

In some embodiments in the step 204, the first parameter comprises any of the parameter types described above with respect to the step 102 an d the details therein will be understood to apply equally to step 204.

In some embodiments, the step 206 of receiving an update to the weight comprises receiving a change in value that should be applied to the weight. In some embodiments, the step 206 may comprise receiving a new value for the weight.

In some embodiments, the step 208 of updating the weight in the first neural network may comprise copying a received value onto the appropriate part of the first neural network. In some embodiments, the step 208 may comprise adjusting the weight (for example, by adding or subtracting an indicated change to the weight from the current value of the weight).

It will be appreciated that the method 200 may be performed with respect to any number of local instances of a neural network. For example, the step of training 202 may comprise training first and subsequent neural networks on first and subsequent sets of medical data. The step 204 may comprise sending a first parameter indicative of a weight from each of the first and subsequent neural networks to a central processor, each first parameter being obtained during training of the respective one of the first or subsequent neural networks. The step of receiving 206 may comprise, receiving at each of the first and subsequent neural networks, an update to the weight from the central processor, the update comprising a combination of the first parameters from each of the first and subsequent neural networks. The step of updating 208 may then comprise updating the weight in each of the first and subsequent neural networks, based on the received update. In this way, learning insights from a plurality of neural networks, each trained on a local set of medical data, may be shared between the plurality of neural networks to speed up the learning process and improve accuracy.

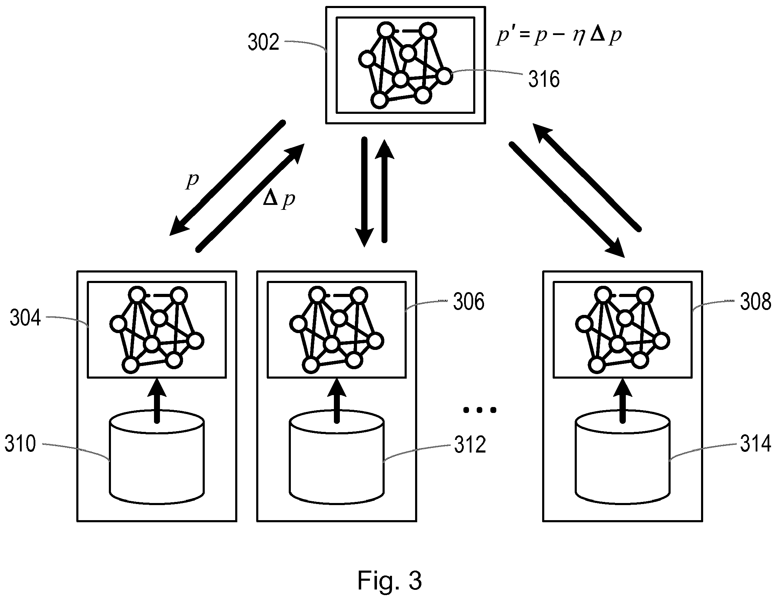

These principles are illustrated with respect to Figure 3 which shows a system for training a neural network according to an embodiment. The system of this embodiment comprises a central parameter server 302, a first neural network 304, a second neural network 306, and a third neural network 308. The central parameter server 302 comprises a processor configured to perform the method 100 above. The central parameter server further comprises a central neural network model 316 (in some embodiments, the central neural network model may be thought of as a master copy of the neural network models, e.g. a copy comprising at any time, the most up-to-date model weights). In this embodiment, the method 200 is performed with respect to each of the first, second and third neural networks.

The first, second and third neural networks 304, 306 and 308 each have access to first, second and third sets of medical data 310, 312, 314 respectively. The first second and third sets of medical data comprise confidential information and cannot be combined into a single data set or sent/transmitted between datasets. As such, the first neural network can only access the first set of medical data 310, the second neural network can only access the second set of medical data 312 and the third neural network can only access the third set of medical data 314.

In this embodiment, each neural network 304, 306 and 308 is associated with one or more imaging devices at different clinical sites. The imaging devices update a local image database that contains clinical images and meta-data (e.g. annotations/classifications provided by a human) collected on the individual medical images and this data forms the respective sets of medical data 310, 312 and 314.

At the beginning of the learning process, an initial copy of the same neural network model (e.g. a copy of the central model described above with respect to method 100), including model parameters *p* (e.g. the weights for the model) is sent from the central parameter server 302 to the three local instances. The central neural network model may be pre-trained with a set of general medical image data, which may, for example, be non-confidential and therefore readily available to the central parameter server 302.

Each of the first, second and third neural networks 304, 306, 308 are then trained on first, second and third sets of medical data 310, 312 and 314 respectively, according to the step 202 of the method 200 shown in Figure 2, using the locally available training data as input and the meta-data as reference output.

Once trained, the first, second and third neural networks 304, 306, 308 then each send a first parameter *p* indicative of a respective weight in each of the neural networks to the central parameter server 302, according to the step 204 of the method 200 as described above with respect to Figure 2. In this embodiment, the parameter is a gradient, or change in the value of the weight compared to the value of the same weight as stored in the central neural network model 312 on the central parameter server 302.

The central parameter server 302 receives parameters from the first, second and third neural networks 304, 306, 308 according to the step 102 of method 100 as described above and combines them to determine an update to the weight according to step 104 as described above with respect to Figure 1.

The central parameter server may combine the parameters according to the equation *p' = p* - *ηΔp* where *p* represents the value of the weight or parameter indicative of the weight, *η* represents a weighing factor, *Δp* represents the average of the gradient values sent by the first, second and third neural networks 304, 306, 308 for the weight *p*, and *p'* represents the updated weight value. In some embodiments, the weights may be updated sequentially, e.g. by applying the equation above to each received gradient, one after another.

According to step 106, as described above, the central parameter server 302 sends the update to the weight to at least the first neural network 304 and may, in some embodiments, send the update to the weight to the first, second and third (i.e. all) neural networks 304, 306, 308 which update the weight in their respective models according to step 208 of the method 200.

The parameter p' may be recalculated every time a new gradient *Δp* is received, or periodically, for example after a predetermined number of gradients are received.

This process is repeated until a quality criterion is satisfied, as described above with respect to method 100.

Once the training process has reached an accuracy satisfying the quality criterion, the determined weight values may be used to initialize new neural network models that are pre-trained with the determined weight values. Using this system, confidential medical data may be stored locally to where it was collected as only model parameters and/or parameter gradients need to be transferred to the central parameter server 302. This reduces data traffic and overcomes data privacy issues.

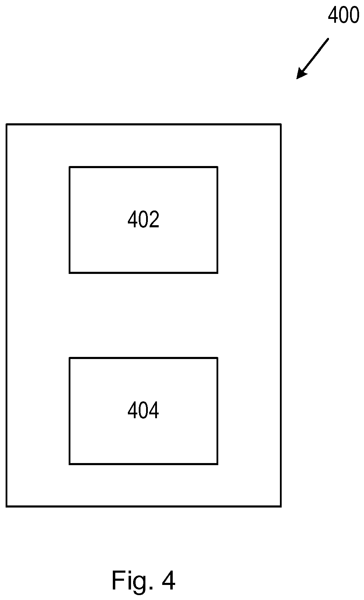

Turning now to Figure 4, according to some embodiments, there is a system 400 configured for training a first neural network to classify medical data. The system 400 comprises a memory 404 comprising instruction data representing a set of instructions. The system 400 further comprises a processor 402 configured to communicate with the memory 404 and to execute the set of instructions. The set of instructions when executed by the processor may cause the processor to perform any of the embodiments of the method 100 as described above. The memory 404 may be configured to store the instruction data in the form of program code that can be executed by the processor 402 to perform the method 100 described above.

In some implementations, the instruction data can comprise a plurality of software and/or hardware modules that are each configured to perform, or are for performing, individual or multiple steps of the method described herein. In some embodiments, the memory 404 may be part of a device that also comprises one or more other components of the system 400 (for example, the processor 402 and/or one or more other components of the system 400). In alternative embodiments, the memory 404 may be part of a separate device to the other components of the system 400.

In some embodiments, the memory 404 may comprise a plurality of sub-memories, each sub-memory being capable of storing a piece of instruction data. In some embodiments where the memory 404 comprises a plurality of sub-memories, instruction data representing the set of instructions may be stored at a single sub-memory. In other embodiments where the memory 404 comprises a plurality of sub-memories, instruction data representing the set of instructions may be stored at multiple sub-memories. Thus, according to some embodiments, the instruction data representing different instructions may be stored at one or more different locations in the system 400. In some embodiments, the memory 404 may be used to store information, such as values of one or more parameters or weights, or other data relevant to calculations made by the processor 402 of the system 400 or from any other components of the system 400.

The processor 402 can comprise one or more processors, processing units, multi-core processors and/or modules that are configured or programmed to control the system 400 in the manner described herein. In some implementations, for example, the processor 402 may comprise a plurality of (for example, interoperated) processors, processing units, multi-core processors and/or modules configured for distributed processing. It will be appreciated by a person skilled in the art that such processors, processing units, multi-core processors and/or modules may be located in different locations and may perform different steps and/or different parts of a single step of the method described herein.

Briefly, the set of instructions, when executed by the processor 402, cause the processor 402 to receive a first parameter indicative of a weight in the first neural network, the first parameter being obtained during training of the first neural network on a first set of medical data. The set of instructions, when executed by the processor 402, further cause the processor 402 to combine the first parameter with a second parameter to determine an update to the weight, the second parameter being indicative of a respective weight in a second neural network. The set of instructions, when executed by the processor 402, further cause the processor to send the update to the weight to the first neural network.

It will be appreciated that the system 400 may comprise additional components to those illustrated in Figure 4. For example, the system 400 may comprise one or more communication interfaces for receiving parameters or other information from the first neural network and/or sending updated parameters or other information to the first neural network. The system 400 may further comprise one or more user interfaces such as a display screen, mouse, keyboard or any other user interface allowing information to be displayed to a user or input to be received from a user. In some embodiments, the system 400 may further comprise a power source such as a battery or mains power connection.

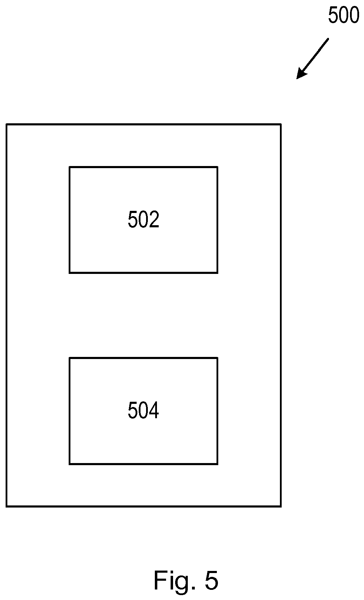

Turning now to Figure 5, Figure 5 shows a system 500 configured for training a first neural network to classify medical data. The system 500 comprises a memory 504 comprising instruction data representing a set of instructions. The system 500 further comprises a processor 502 configured to communicate with the memory 504 and to execute the set of instructions. The set of instructions when executed by the processor may cause the processor to perform any of the embodiments of the method 200 as described above.

Details of processors and memories were described above with respect to processor 402 and memory 404 and the details therein will be understood to apply equally to the processor 502 and the memory 504.

In brief, the set of instructions, when executed by the processor 502, cause the processor 502 to train the first neural network on a first set of medical data, and send a first parameter indicative of a weight in the first neural network to a central processor, the first parameter being obtained during the training of the first neural network. The set of instructions, when executed by the processor 502, further cause the processor 502 to receive an update to the weight from the central processor, the update comprising a combination of the first parameter with a second parameter, the second parameter being indicative of a respective weight in a second neural network, and update the weight in the first neural network, based on the received update.

It will be appreciated that the system 500 may comprise additional components to those illustrated in Figure 5. For example, the system 500 may comprise one or more communication interfaces for sending parameters or other information to the central processor and/or receiving updated parameters or other information from the central processor. The system 500 may further comprise one or more user interfaces such as a display screen, mouse, keyboard or other user interface allowing information to be displayed to a user or input to be received from a user. In some embodiments, the system 500 may further comprise a power source such as a battery or mains power connection.

According to further embodiments, there is also a computer program product comprising a non-transitory computer readable medium, the computer readable medium having computer readable code embodied therein, the computer readable code being configured such that, on execution by a suitable computer or processor, the computer or processor is caused to perform any of the embodiments of methods 100 or 200 as described above.

The term "module", as used herein is intended to include a hardware component, such as a processor or a component of a processor configured to perform a particular function, or a software component, such as a set of instruction data that has a particular function when executed by a processor.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A method performed by a central processor of training a first neural network to classify medical data, the method comprising:
receiving (102) a first parameter indicative of a weight in the first neural network, the first parameter being obtained during training of the first neural network on a first set of medical data;
combining (104) the first parameter with a second parameter to determine an update to the weight, the second parameter being indicative of a respective weight in a second neural network; and
sending (106) the update to the weight to the first neural network.

2. A method as in claim 1 wherein the first neural network and the first set of medical data are located remotely to the central processor; and
wherein the first set of medical data is stored on a separate data storage network or apparatus to the central processor, such that the first set of medical data is inaccessible to the central processor.

3. A method as in claim 1 or 2 wherein the second neural network is trained on a second set of medical data, wherein the second set of medical data is stored on a separate data storage network or apparatus to the central processor and the first neural network, such that the second set of medical data is inaccessible to the central processor and the first neural network.

4. A method as in any one of claims 1 to 3 wherein the first set of medical data comprises confidential data that cannot be transmitted to the central processor or the second neural network due to data privacy.

5. A method as in any one of claims 1 to 4 further comprising:
receiving the second parameter from the second neural network; and/or
sending the update to the weight to the second neural network.

6. A method as in any one of claims 1 to 5 wherein the step of combining (104) comprises taking a weighted average of the first parameter and the second parameter.

7. A method as in any one of claims 1 to 6 further comprising:
repeating the steps of receiving (102), combining (104) and sending (106) until a quality criterion associated with the first neural network is satisfied.

8. A method of training a first neural network to classify medical data, the method comprising:
training (202) the first neural network on a first set of medical data;
sending (204) a first parameter indicative of a weight in the first neural network to a central processor, the first parameter being obtained during the training of the first neural network;
receiving (206) an update to the weight from the central processor, the update comprising a combination of the first parameter with a second parameter, the second parameter being indicative of a respective weight in a second neural network; and
updating (208) the weight in the first neural network, based on the received update.

9. A method as in claim 8 wherein the first set of medical data is stored on a separate data storage network or apparatus to the central processor, such that the first set of medical data is inaccessible to the central processor.

10. A method as in claim 8 or 9 wherein the second neural network is trained on a second set of medical data, wherein the second set of medical data is stored on a separate data storage network or apparatus to the central processor and the first neural network, such that the second set of medical data is inaccessible to the central processor and the first neural network.

11. A method as in claim 8, 9 or 10 wherein the step of training (202) comprises training the first neural network to classify or locate an object in a medical image.

12. A method as in claim 8, 9 or 10 wherein the step of training (202) comprises training the first neural network to determine one or more settings for medical equipment, based on one or more patient attributes.

13. A system (400) configured for training a first neural network to classify medical data, the system comprising:
a memory (404) comprising instruction data representing a set of instructions;
a processor (402) configured to communicate with the memory and to execute the set of instructions, wherein the set of instructions, when executed by the processor, cause the processor to:
receive a first parameter indicative of a weight in the first neural network, the first parameter being obtained during training of the first neural network on a first set of medical data;
combine the first parameter with a second parameter to determine an update to the weight, the second parameter being indicative of a respective weight in a second neural network; and
send the update to the weight to the first neural network.

14. A system (500) configured for training a first neural network to classify medical data, the system comprising:
a memory (504) comprising instruction data representing a set of instructions;
a processor (502) configured to communicate with the memory and to execute the set of instructions, wherein the set of instructions, when executed by the processor, cause the processor to:
train the first neural network on a first set of medical data;
send a first parameter indicative of a weight in the first neural network to a central processor, the first parameter being obtained during the training of the first neural network;
receive an update to the weight from the central processor, the update comprising a combination of the first parameter with a second parameter, the second parameter being indicative of a respective weight in a second neural network; and
update the weight in the first neural network, based on the received update.

15. A computer program product comprising a non-transitory computer readable medium, the computer readable medium having computer readable code embodied therein, the computer readable code being configured such that, on execution by a suitable computer or processor, the computer or processor is caused to perform the method of any one of claims 1 to 12.
